(19) Europäisches Patentamt

European Patent Office

Office européen des brevets

(11) **EP 4 238 645 A1**

(12) **EUROPEAN PATENT APPLICATION**
published in accordance with Art. 153(4) EPC

(43) Date of publication:
06.09.2023 Bulletin 2023/36

(21) Application number: 21886184.7

(22) Date of filing: 26.10.2021

(51) International Patent Classification (IPC):
B01J 20/22 (2006.01)    A61L 9/01 (2006.01)
A61L 9/014 (2006.01)    B01J 20/28 (2006.01)
B01J 20/30 (2006.01)

(52) Cooperative Patent Classification (CPC):
A61L 9/01; A61L 9/014; B01J 20/22; B01J 20/28;
B01J 20/30

(86) International application number:
PCT/JP2021/039438

(87) International publication number:
WO 2022/092066 (05.05.2022 Gazette 2022/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.10.2020 JP 2020182148

(71) Applicant: Toray Industries, Inc.
Tokyo 103-8666 (JP)

(72) Inventors:
• HAYASHI, Toshiki
Otsu-shi, Shiga 520-8558 (JP)
• ASADA, Yasuhiro
Otsu-shi, Shiga 520-8558 (JP)
• MIYOSHI, Kengo
Otsu-shi, Shiga 520-8558 (JP)

(74) Representative: Hoefer & Partner Patentanwälte
mbB
Pilgersheimer Straße 20
81543 München (DE)

(54) **ADHESIVE AGENT, METHOD FOR MANUFACTURING SAME, FILTRATION MATERIAL, AND AIR FILTER**

(57)    An object of the present invention is to provide an adsorbent, a filter medium, and an air filter that have good aldehyde removal performance that undergoes little deterioration over time. The present invention provides an adsorbent in which an inorganic porous medium supports at least an amine-based compound and a compound having a sulfide group as a functional group.

EP 4 238 645 A1

**Description**

TECHNICAL FIELD

The present invention relates to an adsorbent, a filter medium, and an air filter.

BACKGROUND ART

There is an increasing need for improvement in living environment due to an increase in health and comfort consciousness. There are a wide variety of pollutants in indoor air, and among them, aldehyde compounds such as acetaldehyde cause a major problem as pollutants. Acetaldehyde is a typical malodorous component contained in cigarette smoke and exhaust gas from automobiles and has a low odor threshold, which means that the odor is likely to be felt even at a low concentration.

Conventionally, activated carbon having a large surface area and a large pore volume is commonly used for removing malodorous components in the air, but the equilibrium adsorption amount of a lower aliphatic aldehyde to the activated carbon is significantly smaller than that of other malodorous components, and practical performance is not provided.

As a technique for removing a lower aliphatic aldehyde, a method has been proposed in which activated carbon is impregnated with an amine compound to improve the performance thereof (see Patent Document 1).

On the other hand, a conventional adsorbent has had the problem that such amine compounds are easily oxidized by oxygen in the air, whereby the effectiveness of the chemical adsorption action for aldehyde is reduced, and the aldehyde removal performance cannot be maintained for a long period of time.

In order to solve this problem, a technique has been proposed in which silica gel is impregnated with an acid hydrazide compound as an amine compound and a compound having a thiol group as a functional group as an antioxidant to suppress oxidation of an amine-based compound and to suppress deterioration over time of aldehyde removal performance (see Patent Document 2).

PRIOR ART DOCUMENTS

PATENT DOCUMENTS

   Patent Document 1: Japanese Patent Laid-open Publication No. H5-317703
   Patent Document 2: International Publication No. 2015/037483

SUMMARY OF THE INVENTION

PROBLEMS TO BE SOLVED BY THE INVENTION

However, an adsorbent in which silica gel is impregnated with a compound having a thiol group as a functional group has been confirmed to have performance of suppressing deterioration over time, but further improvement in performance has been desired. Accordingly, an object of the present invention is to provide an adsorbent, a filter medium, and an air filter that have good aldehyde removal performance that undergoes little deterioration over time.

SOLUTIONS TO THE PROBLEMS

The present invention provides an adsorbent in which an inorganic porous medium supports at least an amine-based compound and a compound having a sulfide group as a functional group.

The present invention also provides a filter medium including the adsorbent of the present invention.

The present invention also provides an air filter including the filter medium of the present invention.

The present invention also provides a method for manufacturing an adsorbent including dissolving a compound having a sulfide group as a functional group and an amine-based compound in water, impregnating an inorganic porous medium with the solution, and drying the resultant.

EFFECTS OF THE INVENTION

The present invention can provide an adsorbent, a filter medium, and an air filter that have good aldehyde removal performance that undergoes little deterioration over time by impregnating an inorganic porous medium with at least an amine-based compound and a compound having a sulfide group as a functional group.

EMBODIMENT OF THE INVENTION

Hereinafter, the present invention will be described in detail. In the present invention, "or more" means the same as or more than the numerical value indicated therein. Further, "or less" means the same as or less than the numerical value indicated therein.

The adsorbent of the present invention includes an inorganic porous medium. By using the inorganic porous medium, a surface area that can come into contact with treatment air can be secured, and a sufficient amount of an agent described later can be supported, so that the aldehyde removal efficiency can be enhanced.

As the inorganic porous medium employed in the present invention, one selected from the group consisting of activated carbon, zeolite, activated alumina, silica gel, activated clay, aluminum silicate, and magnesium silicate can be preferably used. Two or more selected from the group can be used in combination.

Among the inorganic porous media, porous silica is excellent in that it does not react with an amine-based compound described later and that deterioration of the amine-based compound supported on the porous silica can be suppressed. In addition, the inorganic porous medium is preferably porous silica also from the viewpoint that the porous silica is highly hydrophilic, has a high affinity for a water-soluble agent such as an amine-based compound, and can further enhance the aldehyde adsorption performance of the adsorbent.

The inorganic porous medium used in the present invention is preferably particulate. In the particulate form, it is possible to effectively achieve both performance and economy. By using a fibrous inorganic porous medium, the specific surface area increases, the contact efficiency with a target gas increases, and the performance (removal efficiency) is improved, but such a medium is expensive.

The average particle diameter of the inorganic porous medium is preferably 1 um or more and 1,000 um or less. The average particle diameter referred to herein refers to a mass average particle diameter defined by "Test methods for activated carbon" in JIS K 1474 (2014). By setting the average particle diameter of the inorganic porous medium to 1,000 um or less, more preferably 600 um or less, the adsorption rate of a lower aliphatic aldehyde gas on the adsorbent can be increased, the manufacture is easy, breakage is less likely to occur because of the good strength, and generation of dust due to breakage can also be suppressed. On the other hand, when the average particle diameter is 50 um or more, more preferably 100 um or more, scattering of the inorganic porous medium can be prevented, and handleability and processability can be improved.

The average pore size of the inorganic porous medium in the present invention is preferably 4 nm or more and 50 nm or less. The average pore size in the present invention means a peak diameter obtained by the BJH method, and more specifically, the average pore size is determined using an adsorption-side isotherm obtained by the nitrogen adsorption method at 77 Kelvin (liquid nitrogen temperature). By setting the average pore size of the inorganic porous medium to 50 nm or less, more preferably 30 nm or less, it is possible to increase the specific surface area of the inorganic porous medium while suppressing a decrease in mechanical strength of the inorganic porous medium, and the low-boiling aldehyde removal performance of the adsorbent is further enhanced. In addition, by setting the average pore size of the inorganic porous medium to 4 nm or more, more preferably 5 nm or more, entry of the amine-based compound and VOC gases into the pores of the granular inorganic porous medium can be promoted.

The specific surface area of the inorganic porous medium employed in the present invention is preferably 30 $m^2$/g or more and 1,000 $m^2$/g or less in terms of BET specific surface area. By setting the specific surface area of the inorganic porous medium to 30 $m^2$/g or more, more preferably 50 $m^2$/g or more, the effective area as a reaction field of the amine-based compound supported on the inorganic porous medium is enhanced, and the reaction rate between the adsorbent and the VOC gas to be removed is enhanced. In addition, by setting the BET specific surface area of the inorganic porous medium to 1,000 $m^2$/g or less, it is possible to suppress a decrease in handleability due to a decrease in mechanical strength of the inorganic porous medium, and it is possible to suppress unintentional adsorption of the VOC gas, which leads to secondary odor generation, on the adsorbent.

For the adsorbent of the present invention, it is important that the amine-based compound is supported on the inorganic porous medium. Aldehyde-based odor materials can be effectively adsorbed by the amine-based compound.

It is possible to use, as such an amine-based compound, a primary amine-based compound such as compounds having amino group(s) including aniline, an acid hydrazide compound, benzylamine, naphthylamine, cyclohexylamine, (iso)propanolamine, ethanolamine, diethylenetriamine, triethylenetetramine, styrene-ethylamine methacrylate, and styrene-amine acrylate, a monomer, an oligomer, a polymer, or a derivative containing an amino group derived from these compounds.

Amine-based compounds other than primary amine-based compounds, such as secondary amine-based compounds, include azole compounds such as 3,5-dimethylpyrazole, 3-methyl-5-pyrazolone, 1,2,3-triazole, 1,2,4-triazole, 3-n-butyl-1,2,4-triazole, 3,5-dimethyl-1,2,4-triazole, and 3,5-di-n-butyl-1,2,4-triazole; azine compounds; secondary amine compounds having alkyl groups such as dipropylamine and dibutylamine; and cyclic secondary amine compounds such as piperidine, piperazine, and pyrrolidine.

In addition, the secondary amine compound preferably has an amide bond or a urea bond in order to prevent re-release of aldehyde compounds. Among them, 1,3-dimethylurea and ethyleneurea are more preferable from the viewpoint of high safety, no generation of amine odor, water solubility, and good processability.

Examples of tertiary amine-based compounds include compounds such as vinylbenzyldimethylamine, vinylbenzyldiethylamine, styrene-diethylamine acrylate, styrene-diethylamine methacrylate, styrene-dimethylamine acrylate, styrene-dimethylamine methacrylate, styrene-ethyldimethylamine methacrylate, styrene-ethyldimethylamine acrylate, styrene-ethyldiethylamine methacrylate, styrene-ethyldiethylamine acrylate, and triethylamine, monomers, oligomers, polymers, and tertiary amine-based compounds derived from these compounds.

Among them, a primary amine-based compound having an amino group is preferable, and among them, an acid hydrazide compound is more preferable, because the desorption suppression performance for high-boiling aldehydes of the adsorbent is further improved.

The acid hydrazide compounds are compounds having an acid hydrazide group represented by -CO-NHNH$_2$ derived from a carboxylic acid and hydrazine, and a nitrogen atom having an unshared electron pair is further bonded to the $\alpha$-position of the hydrazide terminal, which significantly improves the nucleophilic reactivity. The aldehyde compound adsorption performance is considered to be able to be exhibited because the unshared electron pair nucleophilically attacks and reacts with the carbonyl carbon atom of the aldehyde compound and immobilizes the aldehyde compound as a hydrazine derivative.

Among aldehyde compounds, acetaldehyde has an electron-donating alkyl group at the $\alpha$-position of the car-bonyl carbon, thus has low electrophilicity of the carbonyl carbon, and is hardly chemically adsorbed. However, the acid hydrazide compound preferably employed in the adsorbent used in the present invention has high nucleophilic reactivity as described above and therefore exhibits good chemical adsorption performance also for acetaldehyde.

In addition, the acid hydrazide compound is preferably a water-soluble acid hydrazide compound from the viewpoint of ease of support treatment on the inorganic porous medium.

Here, the water-soluble acid hydrazide compound refers to an acid hydrazide compound that is dissolved in water (25°C) in an amount of 0.5 mass% or more.

Examples of the acid hydrazide compound used in the present invention include acid monohydrazides having one acid hydrazide group in the molecule, such as formhydrazide, acetohydrazide, propionic acid hydrazide, and benzoic acid hydrazide, acid dihydrazides having two acid hydrazide groups in the molecule, such as carbodihydrazide, glutamic acid dihydrazide, succinic acid dihydrazide, adipic acid dihydrazide, dodecanedioic acid dihydrazide, fumaric acid dihydrazide, maleic acid dihydrazide, and terephthalic acid dihydrazide, and acid polyhydrazides having three or more acid hydrazide groups in the molecule, such as polyacrylic acid hydrazide.

Among them, carbodihydrazide, succinic acid dihydrazide, and adipic acid dihydrazide are preferably used from the viewpoint of adsorption performance. In addition, by using adipic acid dihydrazide, an excellent effect is exerted particularly on the acetaldehyde adsorption capacity.

The amount of the amine-based compound supported is preferably 0.5 parts by mass or more and 30 parts by mass or less with respect to 100 parts by mass of the inorganic porous medium. By setting the amount of the amine-based compound supported to 0.5 parts by mass or more, more preferably 2.0 parts by mass or more, it is possible to obtain a practical effect of improving the aldehyde compound removal efficiency and the aldehyde compound adsorption capacity. On the other hand, by setting the amount of the amine-based compound supported to 30 parts by mass or less, more preferably 20 parts by mass or less, it is possible to inhibit the amine-based compound from crystallizing and blocking the pores of the inorganic porous medium, which causes a decrease in adsorption rate and powder fall-off.

In addition, in the adsorbent of the present invention, it is important that a compound (hereinafter also referred to as a "sulfide compound") having a sulfide group (-S-) as a functional group is also supported on the inorganic porous medium. By doing so, it is possible to obtain an adsorbent in which deterioration of aldehyde removal performance over time is suppressed. The presence of a metal on the pore surface of the inorganic porous medium and the promotion of the decomposition reaction of the amine compound by the catalytic action of the metal are considered to be a main factor of deterioration of the aldehyde compound removal performance over time, and this tendency is particularly remarkable in the case where the amine-based compound is an acid hydrazide compound. On the other hand, it is considered that the sulfide group has reactivity with a metal and thus can suppress the decomposition reaction as described above. Furthermore, since the sulfide group has a property of being easily oxidized, it is considered that supporting of the sulfide compound has an effect of preventing the amine-based compound having good reactivity with aldehyde compounds from being oxidatively decomposed.

Examples of the sulfide compound used in the present invention include methionine compounds in addition to dimethyl sulfide and diethyl sulfide. Specific examples of the methionine compounds include one or two or more selected from L-methionine, D-methionine, DL-methionine, salts thereof, and methionine derivatives such as esters of carboxy groups and amides of carboxy groups contained in these methionines. Among them, methionine is preferable because it is inexpensive and has good heat resistance. Among them, L-methionine is inexpensive and preferably used because it exists in a large proportion in nature.

The amount of the sulfide compound supported is preferably 0.5 parts by mass or more and 20 parts by mass or less with respect to 100 parts by mass of the inorganic porous medium. When the amount of the sulfide compound supported is 0.5 parts by mass or more, more preferably 1.0 parts by mass or more, the sulfide compound can sufficiently react with the metal component adhering to the pore surface. On the other hand, when the amount is 20 parts by mass or less, more preferably 10 parts by mass or less, it is possible to prevent blocking of the pores of the inorganic porous medium and a decrease in the adsorption rate.

Examples of a method for causing the amine-based compound and the sulfide compound to be supported on the inorganic porous medium include a method in which the amine-based compound and the sulfide compound are dissolved in water, the inorganic porous medium is impregnated with the obtained aqueous solution, and then the inorganic porous medium is dried.

The pH (hydrogen-ion exponent) of an aqueous solution in which 5 g of the gas adsorbent of the present invention has been dispersed in 100 g of water having a temperature of 25°C is preferably 3.0 or more and 7.5 or less. The aldehyde compound removal performance is thus improved. Owing to the pH of the aqueous solution being 7.5 or less, more preferably 6.5 or less, the intermediate produced from the reaction by the nucleophilic attack on the carbonyl carbon atom of the aldehyde compound by the unshared electron pair of the amine-based compound is protonated in the acidic reaction field, whereby the intermediate becomes easy to be dehydrated, so that the immobilization reaction to the derivative proceeds sufficiently. Thanks to the pH of the aqueous solution being 3.0 or more, more preferably 4.0 or more, the activity of the unshared electron pair of the amine-based compound to nucleophilically attack the carbonyl carbon atom of the aldehyde compound can be sufficiently maintained.

The pH of the adsorbent can be adjusted by causing the adsorbent to support at least one acid (hereinafter also referred to as an "organic/inorganic acid") selected from the group consisting of organic acids and inorganic acids. The organic/inorganic acid is preferably an acid that does not itself generate odor.

Specific examples of the organic acid include adipic acid, succinic anhydride, sulfanilic acid, malic acid, citric acid, and amino acids. In the case where the inorganic porous medium is impregnated with adipic acid dihydrazide as the amine-based compound in the form of an aqueous dispersion, adipic acid can be preferably employed. Adipic acid is preferably used because it keeps the balance of the dispersion of adipic acid dihydrazide stably but does not generate odor or exhibit hygroscopicity.

As the inorganic acid, phosphoric acid is preferably used. Phosphoric acid is preferably used because it can form poorly soluble salts with dissolved heavy metals such as iron, which promotes the oxidation of sulfide groups, and can insolubilize the heavy metals.

As a method for causing the adsorbent to support the organic/inorganic acid, in the case where impregnation with the amine-based compound and the sulfide compound is performed in the form of an aqueous dispersion, the organic/inorganic acid is preferably added by being mixed with this aqueous dispersion.

Next, a filter medium of the present invention includes the adsorbent of the present invention.

The filter medium of the present invention is preferably formed by sandwiching the adsorbent of the present invention between sheets (hereinafter also referred to as "air-permeable sheets") having air permeability.

As the air-permeable sheets, fiber structures are preferable, and specific examples thereof include cotton-like materials, knitted/woven fabrics, nonwoven fabrics, paper, and other three-dimensional nets. Laminates of these materials can also be used. By adopting such a structure, it is possible to increase the surface area while ensuring air permeability. From the viewpoint of use as an air filter, a nonwoven fabric is preferably used.

Examples of the fiber forming the air-permeable sheets include natural fibers, synthetic fibers, and inorganic fibers such as glass fibers and metal fibers, and among them, synthetic fibers made of a thermoplastic resin capable of being subjected to melt spinning are preferably used. Examples of the thermoplastic resin that forms the synthetic fibers include polyester, polyamide, polyolefin, acryl, vinylon (polyvinyl alcohol), polystyrene, polyvinyl chloride, polyvinylidene chloride, and polylactic acid, which can be selected according to the application and the like. A plurality of types can be used in combination.

The fiber diameter of the fibers constituting the air-permeable sheets can be selected according to the target air permeability and dust collection performance in the use as an air filter and is preferably 1 um or more and 2,000 um or less. By setting the fiber diameter to 1 um or more, more preferably 5 um or more, clogging of the surface of the fiber structures with the adsorbent can be prevented, and deterioration of air permeability can be prevented. In addition, by setting the fiber diameter to 2,000 um or less, more preferably 100 um or less, it is possible to prevent a decrease in the supporting ability of the adsorbent due to a decrease in the fiber surface area and a decrease in the contact efficiency with the treatment air.

The basis weight of the air-permeable sheets is preferably 10 $g/m^2$ or more and 500 $g/m^2$ or less. By setting the basis weight to 10 $g/m^2$ or more, sufficient strength to withstand processing for making the adsorbent supported is obtained, and rigidity necessary for maintaining the filter structure when air passes is obtained. In addition, by setting the basis weight to 500 g/m$^2$ or less, more preferably 200 g/m$^2$ or less, the adsorbent can be uniformly supported up to the inside of the sheets having air permeability, and the handleability at the time of secondary processing into a pleated shape or a honeycomb shape is also good.

It is preferable that at least one of the air-permeable sheets is subjected to electret treatment. The electret treatment makes it possible to collect fine dust of submicron size or nano size, which is difficult to remove normally, by electrostatic force.

As the material to form such an electret air-permeable sheet, materials having high electric resistivity, such as polyolefin resins including polypropylene, polyethylene, polystyrene, polybutylene terephthalate, and polytetrafluoroethylene, aromatic polyester resins including polyethylene terephthalate, and polycarbonate resins, are preferably used.

In the filter medium of the present invention, the adsorbent is preferably fixed to the air-permeable sheets using a thermoplastic resin. By using the thermoplastic resin as a binder resin, it is possible to firmly fix the adsorbent to the air-permeable sheets while preventing the adsorbent from being covered with the binder and deteriorating in the function.

As the thermoplastic resin for immobilizing the adsorbent of the present invention on the air-permeable sheets, a thermoplastic resin such as EVA-based, polyester-based, polyamide-based, and low-density polyethylene-based thermoplastic resins can be used.

As a method for immobilizing the adsorbent on the air-permeable sheets, a method is preferably used in which mixed powder of the adsorbent of the present invention and the thermoplastic resin is scattered on an air-permeable sheet, then another air-permeable sheet is further overlaid, and the product is integrated by hot pressing. By adopting this method, it is possible to prevent the surface of the adsorbent of the present invention from being covered with the thermoplastic resin and deteriorating in the function, which is advantageous in terms of the adsorption rate, and the adsorbing ability can be extremely effectively exhibited.

The amount of the adsorbent supported in the filter medium of the present invention is preferably 5 g/m$^2$ or more and 300 g/m$^2$ or less. By setting the supported amount to 5 g/m$^2$ or more, more preferably 10 g/m$^2$ or more, it is possible to obtain a practical effect of improving the aldehyde compound removal efficiency and the adsorption capacity. In addition, by setting the supported amount to 300 g/m$^2$ or less, more preferably 200 g/m$^2$ or less, it is possible to prevent the surface of an adsorbent sheet having air permeability from being clogged with the adsorbent and to suppress a decrease in air permeability.

The filter medium of the present invention can also support granular activated carbon separately from the adsorbent of the present invention. By making the granular activated carbon supported, VOC gases other than the aldehyde gas can be removed, and VOC gases can be generally adsorbed and removed.

An air filter of the present invention includes the filter medium of the present invention.

As the shape thereof, an original planar shape may be employed, but it is a preferable mode to adopt a pleated shape or a honeycomb shape. The pleated shape in the use as a direct flow type filter and the honeycomb shape in the use as a parallel flow type filter each increase the contact area of the treatment air to improve the collection efficiency and simultaneously reduce the pressure loss.

In addition, in a preferable mode, the air filter of the present invention is formed by putting the filter medium of the present invention in a frame from the viewpoint of air treatment efficiency and handleability.

[EXAMPLES]

[Measurement Methods]

(1) pH of Adsorbent (Hydrogen-Ion Exponent)

In 100 g of pure water having a temperature of 25°C, 5 g of the adsorbent was immersed, lightly stirred, and then allowed to stand for 10 minutes, and the pH of the obtained aqueous solution was measured with a pH meter (EcoScan pH 5 manufactured by Lacom). The measurement was performed three times, and the average value was adopted.

(2) Method for Making Amine-Based Compound and Sulfide Compound Supported

The inorganic porous medium was impregnated with an aqueous solution in which the amine-based compound and the sulfide compound were mixed, and the resultant was dried to perform adjustment.

(3) Basis Weights of Adsorbent and Thermoplastic Resin Supported (g/m$^2$)

Mixed powder obtained by mixing and stirring the adsorbent and the thermoplastic resin was scattered on a sheet having air permeability, another sheet having air permeability was then overlaid, hot pressing was performed to perform integration, and the total basis weight was measured. A value obtained by subtracting the basis weight of the two sheets having air permeability from the total basis weight was multiplied by the charged amount ratio of each component to determine the amount of each of the adsorbent and the thermoplastic resin supported with respect to the entire filter medium.

(4) Acetaldehyde Removal Performance

A flat filter medium measuring 12 cm by 12 cm was attached to an experimental duct measuring 10 cm by 10 cm, and air with a temperature of 23°C and a humidity of 50% RH was sent into the duct at a speed of 0.2 m/sec. Further, acetaldehyde (also expressed as $C_2H_4O$) was added at the upstream end from a standard gas cylinder at an upstream concentration of 10 ppm, and the air was sampled on the upstream and downstream sides of the filter medium. The acetaldehyde concentrations on the respective sides over time were measured using an infrared absorption type continuous monitor, and the removal efficiency was calculated using the following equation.

$$Removal\ efficiency\ (\%)\ =\ [(C0\ -\ C)/C0]\ \times\ 100$$

Here,

C0: Upstream acetaldehyde concentration (10 ppm)
C: Downstream acetaldehyde concentration (ppm)

The removal efficiency after 100 seconds from the start of addition of acetaldehyde was taken as the initial removal efficiency. When the initial removal efficiency of acetaldehyde immediately after preparation of the sample was 40% or more, it was regarded as acceptable.
In addition, the removal efficiency after 100 seconds was measured over time, and the total amount of adsorption (mass increase (g) of the flat filter medium) until the removal efficiency decreased to 5% was divided by the duct area (10 cm × 10 cm) and converted to a value per 1 $m^2$, which was evaluated as the adsorption capacity ($g/m^2$).

(5) Test for Deterioration over Time

In an environment in which the temperature was set to 85°C and the humidity was set to 85% RH, a flat gas adsorption sheet was allowed to stand for 3 days after preparation of the sample and then attached to an experimental duct, and acetaldehyde removal performance was evaluated by the method described in (4) above.
In addition, the reduction rate of the adsorption capacity through the test for deterioration over time was calculated using the following equation.

$$Reduction\ rate\ (\%)\ of\ adsorption\ capacity\ =$$
$$[((adsorption\ capacity\ immediately\ after\ sample$$
$$preparation)\ -\ (adsorption\ capacity\ after\ test\ for$$
$$deterioration\ over\ time))/(adsorption\ capacity\ immediately$$
$$after\ sample\ preparation)]\ \times\ 100$$

In the case where the reduction rate of the adsorption capacity through the test for deterioration over time was 50% or less, it was evaluated as being acceptable.

(6) Heat Resistance Test

In an environment in which the temperature was set to 120°C and the humidity was set to 25% RH, a flat gas adsorption sheet was allowed to stand for 1 day after preparation of the sample and then attached to an experimental duct, and acetaldehyde removal performance was evaluated by the method described in (4) above.
In addition, the reduction rate of the adsorption capacity through the heat resistance test was calculated using

the following equation.

$$\text{Reduction rate (\%) of adsorption capacity} =$$
$$[((\text{adsorption capacity immediately after sample}$$
$$\text{preparation}) - (\text{adsorption capacity after heat resistance}$$
$$\text{test}))/(\text{adsorption capacity immediately after sample}$$
$$\text{preparation})] \times 100$$

In the case where the reduction rate of the adsorption capacity through the heat resistance test was 50% or less, it was evaluated as being "good".

[Example 1] (Adsorbent A)

(Inorganic Porous Medium)

As the inorganic porous medium, porous silica (Fuji Silysia Chemical Ltd.) having an average particle diameter of 300 um was used.

(Amine-Based Compound)

Adipic acid dihydrazide (Otsuka Chemical Co., Ltd.) was used.

(Sulfide Compound)

L-Methionine (manufactured by Wakenyaku Co., Ltd.) was used.

(Preparation of Adsorbent)

An aqueous solution obtained by dissolving 5 mass% of the amine-based compound and 2 mass% of the sulfide compound in water was mixed with the inorganic porous medium, and the resultant was dried to prepare an adsorbent A. When 5 g of the adsorbent A was dispersed in 100 g of water, the pH was 6.3.

(Air-permeable Sheet for Upstream Side)

As the air-permeable sheet positioned on the upstream side with respect to the air flow, a chemical bonded nonwoven fabric having a basis weight of 50 g/m$^2$ and including 16.5 mass% of vinylon (polyvinyl alcohol) fibers having a single fiber fineness of 1.5 dtex, 22 mass% of vinylon (polyvinyl alcohol) fibers having a single fiber fineness of 7.1 dtex, 16.5 mass% of polyethylene terephthalate fibers having a single fiber fineness of 2.0 dtex, and 45 mass% of a phosphorus-based flame retardant-containing acrylic resin binder was used.

(Air-permeable Sheet for Downstream Side)

As the air-permeable sheet positioned on the downstream side with respect to the air flow, a charged fiber sheet having a basis weight of 20 g/m$^2$ and including an electret meltblown nonwoven fabric of polypropylene fibers was used.

(Heat Sealing Resin)

Low-density polyethylene (manufactured by Tokyo Printing Ink Mfg. Co., Ltd., melting point: 98 to 104°C) was used.

(Manufacture of Filter Medium)

The adsorbent and the heat sealing resin were mixed at a mass ratio of 2 : 1 and stirred until the mixture became uniform, and the mixture was scattered on the air-permeable sheet for the downstream side, the air-permeable sheet for the upstream side was overlaid thereon, and the product was hot-pressed to produce a filter medium A.

[Example 2] (Adsorbent B)

(Inorganic Porous Medium)

The same one as used in Example 1 (adsorbent A) was used.

(Amine-Based Compound)

The same one as used in Example 1 (adsorbent A) was used.

(Sulfide Compound)

Diethyl sulfide (manufactured by Tokyo Chemical Industry Co., Ltd.) was used.

(Preparation of Adsorbent)

An aqueous solution obtained by dissolving 5 mass% of the amine-based compound and 2 mass% of the sulfide compound in water was mixed with the inorganic porous medium, and the resultant was dried to prepare an adsorbent B. When 5 g of the adsorbent B was dispersed in 100 g of water, the pH was 6.4.

(Manufacture of Filter Medium)

A filter medium B was produced in the same manner as in Example 1 except that the adsorbent B was used as the adsorbent while using the same air-permeable sheet for the upstream side, air-permeable sheet for the downstream side, and heat sealing resin as those used in Example 1.

[Example 3] (Adsorbent C)

(Inorganic Porous Medium)

The same one as used in Example 1 (adsorbent A) was used.

(Amine-Based Compound)

1,3-Dimethylurea (manufactured by Nacalai Tesque, Inc.) was used.

(Sulfide Compound)

The same one as used in Example 1 (adsorbent A) was used.

(Preparation of Adsorbent)

An aqueous solution obtained by dissolving 5 mass% of the amine-based compound and 2 mass% of the sulfide compound in water was mixed with the inorganic porous medium, and the resultant was dried to prepare an adsorbent C. When 5 g of the adsorbent C was dispersed in 100 g of water, the pH was 6.4.

(Manufacture of Filter Medium)

A filter medium C was produced in the same manner as in Example 1 except that the adsorbent C was used as the adsorbent while using the same air-permeable sheet for the upstream side, air-permeable sheet for the downstream side, and heat sealing resin as those used in Example 1.

[Example 4] (Adsorbent D)

(Inorganic Porous Medium)

The same one as used in Example 1 (adsorbent A) was used.

(Amine-Based Compound)

Carbodihydrazide (manufactured by Japan Finechem Company, Inc.) was used.

(Sulfide Compound)

The same one as used in Example 1 (adsorbent A) was used.

(Preparation of Adsorbent)

An aqueous solution obtained by dissolving 5 mass% of the amine-based compound and 2 mass% of the sulfide compound in water was mixed with the inorganic porous medium, and the resultant was dried to prepare an adsorbent D. When 5 g of the adsorbent D was dispersed in 100 g of water, the pH was 6.5.

(Manufacture of Filter Medium)

A filter medium D was produced in the same manner as in Example 1 except that the adsorbent D was used as the adsorbent while using the same air-permeable sheet for the upstream side, air-permeable sheet for the downstream side, and heat sealing resin as those used in Example 1.

[Example 5] (Adsorbent E)

(Inorganic Porous Medium)

The same one as used in Example 1 (adsorbent A) was used.

(Amine-Based Compound)

Succinic acid dihydrazide (manufactured by Japan Finechem Company, Inc.) was used.

(Sulfide Compound)

The same one as used in Example 1 (adsorbent A) was used.

(Preparation of Adsorbent)

An aqueous solution obtained by dissolving 5 mass% of the amine-based compound and 2 mass% of the sulfide compound in water was mixed with the inorganic porous medium, and the resultant was dried to prepare an adsorbent E. When 5 g of the adsorbent E was dispersed in 100 g of water, the pH was 6.4.

(Manufacture of Filter Medium)

A filter medium E was produced in the same manner as in Example 1 except that the adsorbent E was used as the adsorbent while using the same air-permeable sheet for the upstream side, air-permeable sheet for the downstream side, and heat sealing resin as those used in Example 1.

[Example 6] (Adsorbent F)

(Inorganic Porous Medium)

The same one as used in Example 1 (adsorbent A) was used.

(Amine-Based Compound)

Ethyleneurea (manufactured by Nacalai Tesque, Inc.) was used.

(Sulfide Compound)

The same one as used in Example 1 (adsorbent A) was used.

(Preparation of Adsorbent)

An aqueous solution obtained by dissolving 5 mass% of the amine-based compound and 2 mass% of the sulfide compound in water was mixed with the inorganic porous medium, and the resultant was dried to prepare an adsorbent F. When 5 g of the adsorbent F was dispersed in 100 g of water, the pH was 6.5.

(Manufacture of Filter Medium)

A filter medium F was produced in the same manner as in Example 1 except that the adsorbent F was used as the adsorbent while using the same air-permeable sheet for the upstream side, air-permeable sheet for the downstream side, and heat sealing resin as those used in Example 1.

[Comparative Example 1] (Adsorbent G)

(Inorganic Porous Medium)

The same product as for the adsorbent A was used.

(Amine-Based Compound)

The same one as used in Example 1 (adsorbent A) was used.

(Substitution for Sulfide Compound)

The sulfide compound was not used, and L-cysteine (manufactured by Tomo Chemical Co., Ltd.) was used instead.

(Preparation of Adsorbent)

An aqueous solution obtained by dissolving 5 mass% of the amine-based compound and 2 mass% of L-cysteine in water was mixed with the inorganic porous medium, and the resultant was dried to prepare an adsorbent G. When 5 g of the adsorbent G was dispersed in 100 g of water, the pH was 6.5.

(Manufacture of Filter Medium)

A filter medium G was produced in the same manner as in Example 1 except that the adsorbent G was used as the adsorbent while using the same air-permeable sheet for the upstream side, air-permeable sheet for the downstream side, and heat sealing resin as those used in Example 1.

[Comparative Example 2] (Adsorbent H)

(Inorganic Porous Medium)

The same one as used in Example 1 (adsorbent A) was used.

(Amine-Based Compound)

The same one as used in Example 1 (adsorbent A) was used.

(Substitution for Sulfide Compound)

The sulfide compound was not used, and L-α-alanine (manufactured by Nacalai Tesque, Inc.) was used instead.

(Preparation of Adsorbent)

An aqueous solution obtained by dissolving 5 mass% of the amine-based compound and 2 mass% of L-α-alanine in water was mixed with the inorganic porous medium, and the resultant was dried to prepare an adsorbent H. When 5 g of the adsorbent H was dispersed in 100 g of water, the pH was 6.4.

(Manufacture of Filter Medium)

A filter medium H was produced in the same manner as in Example 1 except that the adsorbent H was used as the adsorbent while using the same air-permeable sheet for the upstream side, air-permeable sheet for the downstream side, and heat sealing resin as those used in Example 1.

[Comparative Example 3] (Adsorbent I)

(Inorganic Porous Medium)

The same one as used in Example 1 (adsorbent A) was used.

(Amine-Based Compound)

The same one as used in Example 1 (adsorbent A) was used.

(Substitution for Sulfide Compound)

The sulfide compound was not used, and succinic anhydride (manufactured by Nacalai Tesque, Inc.) was used instead.

(Preparation of Adsorbent)

An aqueous solution obtained by dissolving 5 mass% of the amine-based compound and 2 mass% of succinic anhydride in water was mixed with the inorganic porous medium, and the resultant was dried to prepare an adsorbent I. When 5 g of the adsorbent I was dispersed in 100 g of water, the pH was 6.5.

(Manufacture of Filter Medium)

A filter medium I was produced in the same manner as in Example 1 except that the adsorbent I was used as the adsorbent while using the same air-permeable sheet for the upstream side, air-permeable sheet for the downstream side, and heat sealing resin as those used in Example 1.

[Comparative Example 4] (Adsorbent J)

(Inorganic Porous Medium)

The same one as used in Example 1 (adsorbent A) was used.

(Amine-Based Compound)

The same one as used in Example 1 (adsorbent A) was used.

(Use or Disuse of Sulfide Compound)

No sulfide compound was used.

(Preparation of Adsorbent)

An aqueous solution obtained by dissolving 5 mass% of the amine-based compound in water was mixed with the inorganic porous medium, and the resultant was dried to prepare an adsorbent J. When 5 g of the adsorbent J was dispersed in 100 g of water, the pH was 6.5.

(Manufacture of Filter Medium)

A filter medium J was produced in the same manner as in Example 1 except that the adsorbent J was used as the adsorbent while using the same air-permeable sheet for the upstream side, air-permeable sheet for the downstream side, and heat sealing resin as those used in Example 1.

[Table 1-1]

| | | Example 1 | Example 2 | Example 3 | Example 4 | Example 5 | Example 6 |
|---|---|---|---|---|---|---|---|
| Adsorbent used | | Adsorbent A | Adsorbent B | Adsorbent C | Adsorbent D | Adsorbent E | Adsorbent F |
| Amine-based compound (part by mass) | Adipic acid dihydrazide | 5.0 | 5.0 | - | - | - | - |
| | Carbodihydrazide | - | - | - | 5.0 | - | - |
| | Succinic acid dihydrazide | - | - | - | - | 5.0 | - |
| | Ethyleneurea | - | - | | - | - | 5.0 |
| | 1,3-Dimethylurea | - | - | 5.0 | - | - | - |
| Sulfide compound (part by mass) | L-Methionine | 2.0 | - | 2.0 | 2.0 | 2.0 | 2.0 |
| | Diethyl sulfide | - | 2.0 | - | - | - | - |
| Other additive component (part by mass) | L-Cysteine | - | - | - | - | - | - |
| | L-$\alpha$-Alanine | - | - | - | - | - | - |
| | Succinic anhydride | - | - | - | - | - | - |
| pH of adsorbent | | 6.3 | 6.4 | 6.4 | 6.5 | 6.4 | 6.5 |
| Charged basis weight (g/m$^2$) | Adsorbent | 50 | 50 | 50 | 50 | 50 | 50 |
| | Thermoplastic resin | 25 | 25 | 25 | 25 | 25 | 25 |
| Supported basis weight (g/m$^2$) | Adsorbent | 51 | 51 | 51 | 51 | 51 | 51 |
| | Thermoplastic resin | 26 | 26 | 26 | 26 | 26 | 26 |
| Immediately after production of sample | Initial removal efficiency (%) of $C_2H_4O$ | 51 | 51 | 41 | 40 | 40 | 40 |
| | $C_2H_4O$ adsorption caoacitv (g/m$^2$) | 0.31 | 0.30 | 0.16 | 0.20 | 0.19 | 0.16 |
| After test for deterioration over time | Initial removal efficiency (%) of $C_2H_4O$ | 47 | 42 | 31 | 36 | 34 | 29 |
| | $C_2H_4O$ adsorption capacity (g/m$^2$) | 0.25 | 0.18 | 0.10 | 0.16 | 0.15 | 0.09 |
| | Reduction rate of adsorption capacity (%) | 20 | 40 | 38 | 20 | 21 | 44 |
| After heat resistance test | Initial removal efficiency (%) of $C_2H_4O$ | 51 | 37 | 30 | 38 | 35 | 28 |
| | $C_2H_4O$ adsorption caoacitv (g/m$^2$) | 0.30 | 0.10 | 0.09 | 0.18 | 0.16 | 0.09 |
| | Reduction rate of adsorption capacity (%) | 3 | 67 | 44 | 10 | 16 | 44 |

[Table 1-2]

| | | Comparative Example 1 | Comparative Example 2 | Comparative Example 3 | Comparative Example 4 |
|---|---|---|---|---|---|
| Adsorbent used | | Adsorbent G | Adsorbent H | Adsorbent I | Adsorbent J |
| Amine-based compound (part by mass) | Adipic acid dihydrazide | 5.0 | 5.0 | 5.0 | 5.0 |
| | Carbodihydrazide | - | - | - | - |
| | Succinic acid dihydrazide | | | | |
| | Ethyleneurea | - | - | - | - |
| | 1,3-Dimethylurea | - | - | - | - |
| Sulfide compound (part by mass) | L-Methionine | - | - | - | - |
| | Diethyl sulfide | - | - | - | - |
| Other additive component (part by mass) | L-Cysteine | 2.0 | - | - | - |
| | L-$\alpha$-Alanine | - | 2.0 | - | - |
| | Succinic anhydride | - | - | 2.0 | - |
| pH of adsorbent | | 6.5 | 6.4 | 6.5 | 6.5 |
| Charged basis weight (g/m$^2$) | Adsorbent | 50 | 50 | 50 | 50 |
| | Thermoplastic resin | 25 | 25 | 25 | 25 |
| Supported basis weight (g/m$^2$) | Adsorbent | 51 | 51 | 51 | 51 |
| | Thermoplastic resin | 26 | 26 | 26 | 26 |
| Immediately after production of sample | Initial removal efficiency (%) of $C_2H_4O$ | 51 | 49 | 50 | 50 |
| | $C_2H_4O$ adsorption capacity (g/m$^2$) | 0.30 | 0.29 | 0.29 | 0.30 |
| After test for deterioration over time | Initial removal efficiency (%) of $C_2H_4O$ | 39 | 39 | 20 | 20 |
| | $C_2H_4O$ adsorption capacity (g/m$^2$) | 0.12 | 0.11 | 0.02 | 0.01 |
| | Reduction rate of adsorption capacity (%) | 60 | 63 | 92 | 96 |
| After heat resistance test | Initial removal efficiency (%) of $C_2H_4O$ | 36 | 40 | 36 | 36 |
| | $C_2H_4O$ adsorption capacity (g/m$^2$) | 0.10 | 0.12 | 0.10 | 0.10 |
| | Reduction rate of adsorption capacity (%) | 67 | 59 | 66 | 67 |

<Summary>

The acetaldehyde removal efficiency, the adsorption capacity, and the reduction rate of the adsorption capacity of Examples 1 to 6 and Comparative Examples 1 to 4 are shown in Table 1.

In all of Examples 1 to 6, since a sulfide compound was used in combination with an amine-based compound, the initial removal efficiency of acetaldehyde immediately after preparation of the sample satisfied 40% or more, and the reduction rate of the adsorption capacity after the test for deterioration over time also satisfied 50% or less as compared with Comparative Examples 1 to 4.

In all of Examples 1, 2, 4, and 5, since an acid hydrazide compound was employed as the amine-based compound, the acetaldehyde adsorption capacity immediately after preparation of the sample was higher than those in Examples 3 and 6.

Furthermore, in both of Examples 1 and 2, since adipic acid dihydrazide was employed as the amine-based compound, the acetaldehyde adsorption capacity immediately after preparation of the sample was 0.30 $g/m^2$ or more, and the initial removal efficiency was 50% or more, which were particularly high, as compared with Examples 3 to 6.

Further, in all of Examples 1 and 3 to 6, since L-methionine was employed as the sulfide compound, the reduction rate of the adsorption capacity after the heat resistance test was a low value satisfying 50% or less as compared with Example 2.

INDUSTRIAL APPLICABILITY

The filter medium using the adsorbent according to the present invention is preferably used as a filter material for an air filter for cleaning air in a vehicle interior of an automobile, a railway vehicle, or the like, a filter for an air cleaner used in healthy housing, a pet-compatible apartment, an elderly facility, a hospital, an office, or the like, a filter for an air conditioner, an intake/exhaust filter of an OA device, a filter for a building air conditioner, a filter for an industrial clean room, or the like.

**Claims**

1.  An adsorbent comprising:

    an inorganic porous medium; and
    at least an amine-based compound and a compound having a sulfide group as a functional group supported on the inorganic porous medium.

2.  The adsorbent according to claim 1, wherein the amine-based compound is an acid hydrazide compound.

3.  The adsorbent according to claim 1 or 2, wherein the compound having a sulfide group as the functional group is methionine.

4.  The adsorbent according to any one of claims 1 to 3, having a pH of 3.0 or more and 7.5 or less when 5 g of the adsorbent is dispersed in 100 g of water.

5.  The adsorbent according to any one of claims 1 to 4, further comprising at least one acid selected from the group consisting of other organic acids and inorganic acids, the at least one acid supported on the inorganic porous medium.

6.  The adsorbent according to any one of claims 1 to 5, wherein the inorganic porous medium is porous silica.

7.  A filter medium comprising the adsorbent according to any one of claims 1 to 6.

8.  The filter medium according to claim 7, wherein the adsorbent is sandwiched between sheets having air permeability.

9.  The filter medium according to claim 7 or 8, wherein the adsorbent is sticked to the sheets using a thermoplastic resin.

10.  An air filter comprising the filter medium according to any one of claims 7 to 9.

11.  A method for manufacturing an adsorbent, comprising:

dissolving a compound having a sulfide group as a functional group and an amine-based compound in water;
impregnating an inorganic porous medium with the solution; and
drying the resultant.

## INTERNATIONAL SEARCH REPORT

| International application No. |
| --- |
| **PCT/JP2021/039438** |

### A. CLASSIFICATION OF SUBJECT MATTER

*B01J 20/22*(2006.01)i; *A61L 9/01*(2006.01)i; *A61L 9/014*(2006.01)i; *B01J 20/28*(2006.01)i; *B01J 20/30*(2006.01)i
FI:   B01J20/22 A; B01J20/30; B01J20/28 Z; A61L9/014; A61L9/01 K; A61L9/01 H

According to International Patent Classification (IPC) or to both national classification and IPC

### B. FIELDS SEARCHED

Minimum documentation searched (classification system followed by classification symbols)

B01J20/22; A61L9/01; A61L9/014; B01J20/28; B01J20/30

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus (STN); JSTPlus (JDreamIII); JMEDPlus (JDreamIII); JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| A | WO 2016/167258 A1 (OSAKA GAS CHEMICALS CO., LTD.) 20 October 2016 (2016-10-20) | 1-11 |
| A | JP 2018-114463 A (OSAKA GAS CHEMICALS CO., LTD.) 26 July 2018 (2018-07-26) | 1-11 |
| A | WO 2015/037483 A1 (TORAY IND., INC.) 19 March 2015 (2015-03-19) | 1-11 |
| A | JP 2016-040033 A (TOKYO OHKA KOGYO CO., LTD.) 24 March 2016 (2016-03-24) | 1-11 |
| A | WO 2014/092133 A1 (TOSOH CORP.) 19 June 2014 (2014-06-19) | 1-11 |
| A | WO 2012/091125 A1 (TOSOH CORP.) 05 July 2012 (2012-07-05) | 1-11 |
| A | JP 2019-155290 A (OSAKA SODA CO., LTD.) 19 September 2019 (2019-09-19) | 1-11 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **03 December 2021** | **21 December 2021** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2021/039438**

| Patent document cited in search report | | | Publication date (day/month/year) | Patent family member(s) | | | Publication date (day/month/year) |
|---|---|---|---|---|---|---|---|
| WO | 2016/167258 | A1 | 20 October 2016 | (Family: none) | | | |
| JP | 2018-114463 | A | 26 July 2018 | (Family: none) | | | |
| WO | 2015/037483 | A1 | 19 March 2015 | EP | 3045224 | A1 | |
| | | | | CN | 105517702 | A | |
| | | | | KR | 10-2016-0054467 | A | |
| JP | 2016-040033 | A | 24 March 2016 | US | 2017/0216814 | A1 | |
| | | | | WO | 2016/024541 | A1 | |
| | | | | TW | 201622810 | A | |
| | | | | KR | 10-2017-0041740 | A | |
| WO | 2014/092133 | A1 | 19 June 2014 | JP | 2014-133227 | A | |
| | | | | JP | 2014-140835 | A | |
| WO | 2012/091125 | A1 | 05 July 2012 | JP | 2012-171939 | A | |
| | | | | JP | 2012-149344 | A | |
| | | | | US | 2013/0281726 | A1 | |
| | | | | EP | 2659964 | A1 | |
| | | | | CN | 103282116 | A | |
| JP | 2019-155290 | A | 19 September 2019 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H5317703 A

- WO 2015037483 A